# EUROPEAN PATENT APPLICATION

(11) **EP 3 153 122 A2**
(43) Date of publication of application: **12.04.2017**
(21) Application number: 16275138.2
(22) Date of filing: 20.09.2016
(51) Int. Cl.: A61B 18/08, A61B 34/20

(54) **APPARATUS AND METHOD FOR OCCLUDING A VESSEL**

(30) Priority: 08.10.2015 GB 201517822
(71) Applicant: Cook Medical Technologies LLC, Bloomington, IN 47404 (US)
(72) Inventor: Elgaard, Per, 4690 Haslev (DK); Torp, Allan, 4632 Bjaeverskov (DK)
(74) Representative: Williams Powell

(57) **Abstract**

An ablation system (10) includes an electrical ablation element (12) of elongate form with an electrically conductive element including a resistive part (22). A control unit (40) includes a processing unit (42), a power unit (44) and, optionally, a temperature sensor (46). The control unit (40) in one embodiment carries out ablation in at least two phases, the first phase at a higher energy level and a second phase, after at least partial retraction of the resistive part (22), at a second lower phase in order to close any remaining lumen within a blood flow obstruction formed within the vessel (18) during the first phase. Other embodiments provide for sensing retraction of the resistive part (22) and effecting ablation during and/or after the retraction process in order to create a more effective occlusion barrier.

## Description

### Technical Field

The present invention relates to apparatus and a method for occluding or closing a vessel.

### Background Art

There are numerous medical conditions when it is desired or necessary to close a body vessel, including for instance in the treatment of aneurysms, arteriovenous malformations, arteriovenous fistulas, for starving organs of oxygen and nutrients for instance in the treatment or containment of cancerous growths, and so on.

Several techniques are known and in use for closing or occluding such body vessels. Traditionally, vessels have been closed by means of external ligation, which generally must be carried out by an open surgery procedure, with its associated risks, inconvenience and long patient recovery times. Other more recent methods aim to use an endoluminal procedure to insert into the vessel or organ one or more occlusion devices, such as a metal framed occluder, coils, pellets or the like, able to obstruct the flow of blood in the vessel.

It is also known to seek to constrict a vessel by endoluminal ablation, causing contraction of the vessel and/or coagulation of blood to form a blood clot in the vessel. A technique which has been considered suitable is RF ablation, in which an electrical terminal is fed endoluminally into the vessel and an electrical pulse at RF frequencies applied to the electrical terminal. The conductivity of blood and/or the vessel tissues causes localised heating. This heating can be used to cause damage to the tissue (intima) of the vessel wall, resulting in vessel contraction. In other devices RF ablation heats the surrounding blood, causing this to coagulate around the electrical terminal and form a blood clot which blocks the vessel.

Two types of RF ablation apparatus are generally contemplated in the art, the first being a monopolar system having an elongate active terminal and a passive/dispersive pad. The active terminal is designed to be fed endoluminally into the patient's vessel, while the passive pad is positioned against the person's outer body, as close as practicable to the active terminal. Electrical energy applied to the active terminal will pass by conduction through the patient to the passive pad. There will be localised heating at the active terminal, which effects the desired ablation.

A problem with monopolar systems is that it can be difficult to control the extent of damage to surrounding tissues and organs, as well as to the vessel wall. This risks damaging the vessel to the point of rupture, as well as possible irreversible damage to neighbouring organs.

Another RF ablation system uses a bipolar arrangement, in which an elongate electrical element includes two terminals, which are spaced longitudinally from one another at a distal end of the electrical element. Current passes between the two terminals through the surrounding blood, causing localised heating and coagulation of the blood. A bipolar system has been considered to provide more localised heating and therefore reduced risk of damage to surrounding organs and tissue.

A problem with both systems lies with the retraction of the electrical terminal from the vessel at the end of the ablation process. In a system which ablates the vessel wall to cause its contraction, the electrical terminal can become attached to the vessel wall tissue, with the risk of tearing and rupturing the vessel wall. In a system which ablates the surrounding blood to generate a blood clot in the vessel, there is the risk that the blood clot is dragged with the electrical element and that the occlusion of the vessel is as a consequence lost. There is also the risk of leaving an opening in blood clot where the electrical terminal resided, which can result in incomplete occlusion and the risk of recanalization.

Some such devices have attempted to address the above problems by having an electrical element with a detachable terminal end. However, this entails leaving a foreign body in the patient.

Examples of prior art devices and methods can, for instance, be found in US-2009/0248007, US-2001/0020167, US-2001/0016739, US-6,539,265, WO-2010/080974, US-6,264,650, US-6,066,139, US-6,676,657, US-2010/0268217, US-5,709,224, US-6,398,779, US-6,019,757 and US-5,743,905.

### Summary of the Invention

The present invention seeks to provide improved apparatus for occluding or closing a body vessel.

According to an aspect of the present invention, there is provided apparatus for closing a blood vessel including:
an electrically conductive element for being passed endoluminally to a treatment site, the electrically conductive element having a proximal section and a distal section, having first and second power supply coupling zones at the proximal section, and having a resistive part positionable at the distal section, wherein the electrically conductive element electrically couples the first and second power supply coupling zones via the resistive part;
a power supply for coupling to the first and second power supply coupling zones for supplying energy to the resistive part;
a detection unit for detecting at least one operational change at the resistive part;
a control unit coupled to the power supply and to the detection unit; wherein the control unit is operable to control the power supply to supply energy to the resistive part at a first power level, to detect at least one operational change at the resistive part and to control the power supply to supply energy to the resistive part at a second power level lower than the first power level once the operational change has been detected.

The apparatus can be controlled to cause a primary blood flow obstruction, for example including a primary blood coagulation and/or a vessel contraction, when operated at the first power level, which can create a vessel occluding barrier.

The apparatus can be controlled to cause a secondary blood flow obstruction, for example including a secondary blood coagulation, when operated at the second power level.

Preferred embodiments provide heat induced vessel embolization and vessel contraction using resistive heating.

The apparatus disclosed herein provides for closing any opening or lumen left by a retracting or retracted electrical ablation element or electrically conductive element, namely by applying energy through the electrically conductive element, at a power level less than the initial ablation power, which has the effect of causing secondary blood coagulation. Preferably, the second power level is insufficient to cause the creation of a further vessel occluding barrier, that is a second barrier which closes off the vessel in its entirety. Specifically, the application of the power at the second level stops once sufficient blood has coagulated to close any aperture left by the retracted or retracting electrical ablation element or electrically conductive element. The occluding barrier created by application of power at the first power level will therefore form the total length of effective vessel occlusion, thereby making the method also suitable in vessel zones having short treatment sites, such as in locations with adjacent vessel side branches and the like.

The resistive part could be said to be a resistive heating element. In embodiments, the power supply is for coupling to the first and second power supply coupling zones to form a closed loop including the resistive part.

Preferably, the apparatus includes a user notification unit coupled to the control unit, the control unit being operable to generate a notification on detection of the operational change in the resistive part. The notification unit could be a visual notification, an acoustic notification, a vibratory notification, a combination of any of these, or any other suitable notification.

Preferably, the control unit is operable to command a partial retraction of the resistive part in a proximal direction on detection of the operational change. Partial retraction may leave a part of the resistive part within the formed blood flow obstruction or clot, thereby to keep any aperture therein closed and to ensure that the second phase of ablation or embolization takes place within the blood flow obstruction or clot and able to close off the residual lumen.

In an embodiment, the apparatus may include a positioning, or drive, unit coupled to the control unit, the positioning unit being operable to effect the partial retraction of the resistive part in the proximal direction on detection of the operational change. The control unit may be operable to generate a notification to effect said partial retraction of the resistive part.

There may be provided a resistive part position sensor coupled to the control unit, wherein the control unit is operable to control the power supply to supply energy to the resistive part at the or a second power level lower than the first power level when partial retraction of the resistive part has been detected.

In some embodiments the control unit may be operable to command the power supply to supply power to the resistive part until the resistive part has been retracted by a predetermined distance. The predetermined distance may be equivalent to a desired length of closure of the vessel. In other words, the system of this embodiment is able to create an occluding barrier of varying length.

The operational change for any of the embodiments herein can include a change in impedance, inductance, capacitance, resistivity, temperature, adhesion for example to target tissues, pressure, or position.

There may be provided a temperature sensor and/or a pressure sensor at the distal section of the electrically conductive element and/or at the resistive part. In some embodiments, the temperature sensor can be provided by the resistive part itself where the resistive part has thermosensing properties as in a thermistor.

According to another aspect of the present invention, there is provided apparatus for closing a blood vessel including:
an electrically conductive element for being passed endoluminally to a treatment site, the electrically conductive element having a proximal section and a distal section, having first and second power supply coupling zones at the proximal section, and having a resistive part positionable at the distal section, wherein the electrically conductive element electrically couples the first and second power supply coupling zones via the resistive part;
a power supply for coupling to the first and second power supply coupling zones for supplying energy to the resistive part;
a position sensor arranged to detect the position of the resistive part in a patient;
a control unit coupled to the power supply and to the position sensor; wherein the control unit is operable to control the power supply to supply energy to the resistive part at a first power level, to determine movement of the resistive part and to apply power to the resistive part when the resistive part has been deemed to have been moved by a first predetermined distance.

In this aspect, the system monitors for a change in the position of the resistive part and applies ablation energy in a manner which can elongate the occlusive barrier which is produced.

In one embodiment, the control unit is operable to apply power to the resistive part continuously when in an initial position and while the resistive part is moved by the predetermined distance. The control unit may be operable to apply power at the first power level in the initial position and at the first or second power level during retraction. In another embodiment, the control unit is operable to apply power to the resistive part discontinuously, when in an initial position and after the resistive part has been moved by the predetermined distance.

Preferably, the control unit is operable to apply power to the resistive part up to a second distance greater than the first predetermined distance. The second distance may be equivalent to a desired length of closure of the vessel. The control unit may be operable to repeat, up to the second distance, the steps of supplying energy to the resistive part at the first power level, determining movement of the resistive part by the first predetermined distance, and applying power to the resistive part when the resistive part has been deemed to have been moved by the first predetermined distance.

Advantageously, the resistive part has a longitudinal length and the first predetermined distance is less than said longitudinal length.

In an embodiment, the apparatus includes a detection unit for detecting at least one operational change at the resistive part, the detection unit being coupled to the control unit. The control unit can be operable to control the power supply to supply energy to the resistive part at a second power level lower than the first power level once the operational change has been detected. This is not essential though, and in other embodiments the control unit may be operable to apply power at the first power level or near the first power level so as to produce an occlusive barrier of radial dimensions at least as significant as that produced prior to movement of the resistive part. The control unit may be operable to command retraction of the resistive part by the first predetermined distance on detection of the operational change.

After the resistive part has been moved by the first predetermined distance, the control unit can be operable to apply power to the resistive part at the first power level.

There may be provided a positioning unit coupled to the control unit, the positioning unit being operable to move the resistive part in at least a proximal direction. Such a positioning unit can therefore provide an automated ablation system.

According to another aspect of the present invention, there is provided a method of closing a blood vessel by means of apparatus including an electrically conductive element having a resistive part positionable at a distal section thereof and a power supply for supplying energy to the resistive part; the method including the steps of: supplying energy to the resistive part at a first power level; detecting at least one operational change at the resistive part; when said operational change has been detected supplying energy to the resistive part at a second power level lower than the first power level.

The method can include the step of generating a user notification on detection of the operational change in the resistive part.

The method can include the step of partially retracting the resistive part in a proximal direction on detection of the operational change.

The method can include the step of detecting partial retraction of the resistive part.

The resistive part can be retracted by a predetermined distance while supplying power thereto. The predetermined distance can be equivalent to a desired length of closure of the vessel.

The method can include the step of detecting partial retraction of the resistive part, and can include supplying energy to the resistive part at the second power level when partial retraction of the resistive part has been detected.

Operating the apparatus at the second power level can include causing a blood coagulation in a vessel.

According to an aspect of the invention, there is provided a method of closing a blood vessel by means of apparatus including an electrically conductive element having a resistive part positionable at a distal section thereof and a power supply for supplying energy to the resistive part; the method including the steps of: determining movement of the resistive part; applying power to the resistive part when the resistive part has been deemed to have been moved by a first predetermined distance.

The method can include applying power to the resistive part continuously when in an initial position and while the resistive part is moved by the predetermined distance; or including applying power to the resistive part discontinuously, when in an initial position and after the resistive part has been moved by the predetermined distance. Applying power continuously can include causing a blood flow obstruction, provided at least in part by a blood coagulation in a vessel, while the resistive part is moved by the predetermined distance. Power may be applied at the first power level in the initial position and at the first or second power level during retraction.

The method can include applying power to the resistive part up to a second distance greater than the first predetermined distance.

The method can include detecting at least one operational change at the resistive part. The method can include supplying energy to the resistive part at a second power level lower than the first power level once the operational change has been detected. The method can include commanding retraction of the resistive part by the first predetermined distance on detection of the operational change.

After the resistive part has been moved by the first predetermined distance, the method can include applying power to the resistive part at the first power level.

The method may include repeating, up to the second distance, the steps of supplying energy to the resistive part at the first power level, determining movement of the resistive part by the first predetermined distance, and applying power to the resistive part when the resistive part has been deemed to have been moved by the first predetermined distance.

The method may include steps appropriate for effecting the functionality disclosed herein.

The apparatus of the method may include any of the features of the apparatus described herein.

The electrically conductive element can be for being passed endoluminally to a treatment site.

Supplying energy to the resistive part at the first power level can be to cause a primary blood flow obstruction, which may be a combination of vessel contraction and a primary blood coagulation.

Supplying energy to the resistive part at the second power level can be to cause a secondary blood flow obstruction which may be a secondary blood coagulation.

Other features of the apparatus and method disclosed herein will become apparent from the following specific description of preferred embodiments.

### Brief Description of the Drawings

Embodiments of the present invention are described below, by way of example only, with reference to the accompanying drawings, in which:
Figure 1A is a schematic diagram of an embodiment of an ablation system;
Figure 1B is a schematic diagram of an embodiment of an ablation system in operation in a vessel;
Figures 2A to 2D and 3A to 3F depict the ablation process of the preferred embodiment;
Figures 4 to 6 are flow charts depicting different functionalities of the control system and preferred steps of operating the apparatus;
Figure 7 is a schematic diagram of an embodiment of an ablation system;
Figure 7A is a schematic diagram of an embodiment of an ablation system;
Figure 8 shows an embodiment of an ablation system; and
Figure 9 shows an embodiment of an ablation system.

### Description of the Preferred Embodiments

There are described below various embodiments of apparatus and methods for effecting ablation, which may include embolization, of a body vessel, in particular a blood vessel. The preferred embodiments are designed to create blood clotting, that is to embolize or ablate the blood surrounding the operative part of the apparatus, as well as some contraction of the vessel. This can be achieved by selecting an ablation energy level and an ablation time duration suitable to heat surrounding blood and the vessel wall. The skilled person will be able to determine suitable ablation parameters from common general knowledge in the art. Moreover, the preferred embodiment uses a thin, that is narrow diameter, operative part to be able to navigate in the vessels to selective anatomical targets.

It is to be appreciated that the level of power applied through the operative part and the time of application will be dependent upon factors including the size of the vessel, the amount and speed of blood flow through the vessel, pulsation and turbulence of blood at the point of ablation, and so on. For small vessels the energy will typically be 10-50W and the time 10 to 60 seconds.

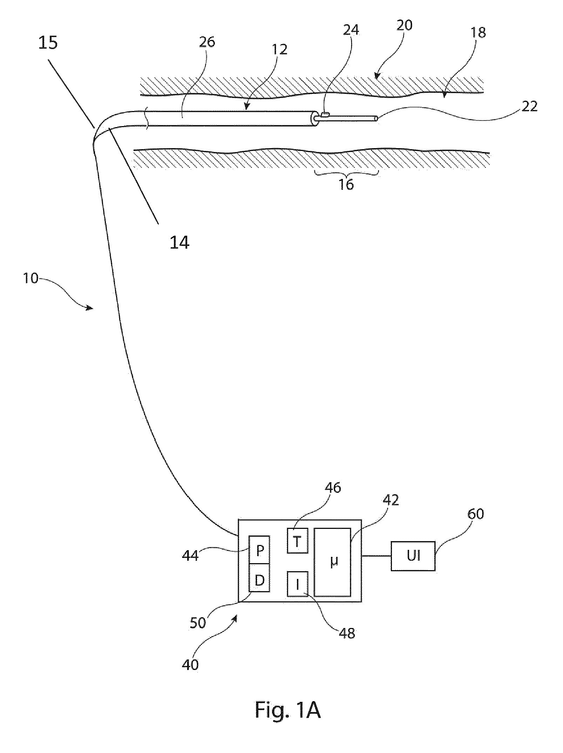

Referring first to Figure 1A, this shows in schematic form an embodiment of an ablation system 10 having an electrical ablation element 12 including an electrically conductive element both arranged in an elongate manner along a longitudinal axis of the apparatus. The electrical ablation element 12 is designed to be passed endoluminally through the vasculature of a patient up to a treatment site 16 of a vessel 18, that is to the position in a vessel at which it is desired to close or occlude the vessel 18.

The electrically conductive element 12 includes a proximal section 19 and a distal section 20. The proximal section in this embodiment includes first and second power supply coupling zones 14, 15, and the electrically conductive element electrically couples the first and second power supply coupling zones 14, 15 via a resistive part 22 at the distal section 20. For example, the electrically conductive element can include a loop of wire or other conductive element from the first power supply coupling zone 14, via the distal section of the electrically conductive element, where it forms the resistive part 22, and to the second power supply coupling zone 15. In another example, the conductive element may include a first conductive isolated wire coupling a first end of a resistive heating element to the first power supply coupling zone 14 and a second conductive isolated wire coupling a second end of the resistive heating element to the second power supply coupling zone 15.

The resistive part 22 has a higher electrical resistance (e.g. 50-200 ohms) than the other parts of the electrically conductive element. In this embodiment, the resistive part is the operative part of the system. The resistive part 22 is configured so that the application of power to the power supply coupling zones causes current to flow through the resistive part which can cause heating of the resistive part, whereby to cause heating, and therefore embolization or ablation, of blood surrounding the resistive part, and/or heating and consequential contraction of the vessel in the vicinity of the resistive part 22.

As can be seen in Figure 1A, the electrical ablation element 12 in this embodiment also includes a temperature sensor 24 for measuring temperature at the resistive part 22, useful in determining the progress of ablation of the vessel.

The electrical ablation element 12 also includes a sheath 26 of electrically insulating material which in practice covers the remainder of the electrically conductive element other than the resistive part. In other words, the resistive part 22 is exposed beyond the end of the sheath so as to be in direct contact with blood when inserted into a lumen. However, this is not to say that the resistive part is necessarily electrically exposed although it can be in some embodiments. In this embodiment the resistive part is electrically isolated. The resistive part can be electrically insulated by means of a polymer coating such as PTFE.

In this embodiment, the electrically conductive element within the sheath 26 has a lower electrical resistance than the exposed resistive part 22 whereby to prevent excessive heating of the electrically conductive element within the sheath 26.

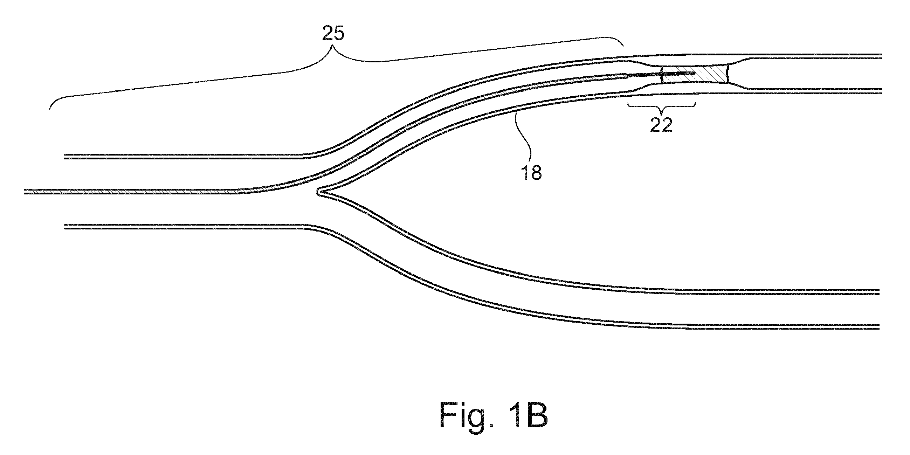

As can be seen in Figure 1B, which shows the electrical ablation element in a vessel 18, the part of the apparatus in which the sheath 26 covers the electrically conductive element can be referred to as a proximal isolated part 25 of the device. The exposed resistive part 22 can be referred to as an active part of the device.

The apparatus can be said to have an elongate section including the section to be inserted into a patient. The longitudinal length of the elongate section of the apparatus in this embodiment is about 1.5 metres, and the diameter of the apparatus in the elongate section is preferably in the region of 0.5 to 1 mm.

Referring again to Figure 1A the electrically conductive element is coupled electrically to a control unit 40. In particular, the control unit 40 is coupled to the first and second power supply coupling zones 14, 15. As explained below, the control unit 40 is operable to provide energy to the electrically conductive element by applying a potential difference between the first and second power supply coupling zones 14, 15, which will drive current through the resistive part 22. The current provided can be alternating or direct current. However, if it is alternating current, it is preferably at a frequency of at least 100 kHz to avoid stimulating a patient's nerves or muscles. The resistive part 22, owing to its high resistance, will be caused to heat as a result of the current passing through it, and as a result there will be consequential heating of blood and in some scenarios also of the vessel wall in the vicinity of the resistive part 22, as explained in further detail below.

The control unit 40 typically includes a processing unit 42, a power delivery circuit 44 coupled to the first and second power supply coupling zones 14, 15 of the system 10, typically one or more sensors including a temperature sensor unit 46 coupled to the temperature probe 24 of the system 10 and/or an impedance sensor unit 48. In some embodiments both of types of sensor units 46 and 48, and/or other sensor units as described elsewhere herein, may be provided.

The control unit 40 may also include a resistive part drive unit 50 for moving the resistive part 22, in this embodiment for moving the entire electrically conductive element or electrical ablation element 12, within the patient's vessel 18. In some embodiments, the control unit 40 may be provided with a position sensor for measuring the position of the resistive part 22 or the electrically conductive element or electrical ablation element 12 within a patient and in particular for measuring the retraction of the resistive part 22 or the electrically conductive or electrical ablation element 12 from within the vessel 18. Some embodiments may include both a drive unit 50 and a position sensor.

The processing unit 40 also includes a user interface 60 coupled to the control unit 40 and operable to provide data to a user and for input of user commands to the control unit 40. The user interface 60 may, in its simplest embodiment, include an on/off switch for operating the control unit 40 and therefore the ablation, with the control unit 40 then effecting the desired ablation process under the command of the unit 40 solely. In other embodiments, the user interface 60 may be more sophisticated and enable, for example, a user to select different modes of ablation and also to produce, for instance, occluding barriers of different lengths, as described in further detail below.

The user interface 60 preferably also includes an output for providing ablation feedback and/or warning signals to a user. It may, for example, provide an indication of measured temperature, an indication of progress of ablation of the vessel and so on. For such purposes, the user interface 60 may include a visual unit, for example a display to display quantitative data such as graphs, measures of temperature, determined length of occlusion and so on. In other embodiments the display may be simpler, having for instance simple visual indicators such as one or more illuminated lamps. The output could also be an acoustic output and/or, as appropriate, a tactile output such as a vibration generator and so on. Any combination of user feedback devices may be provided.

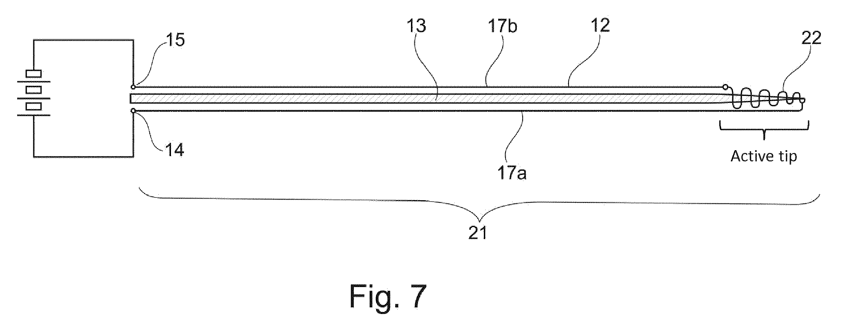

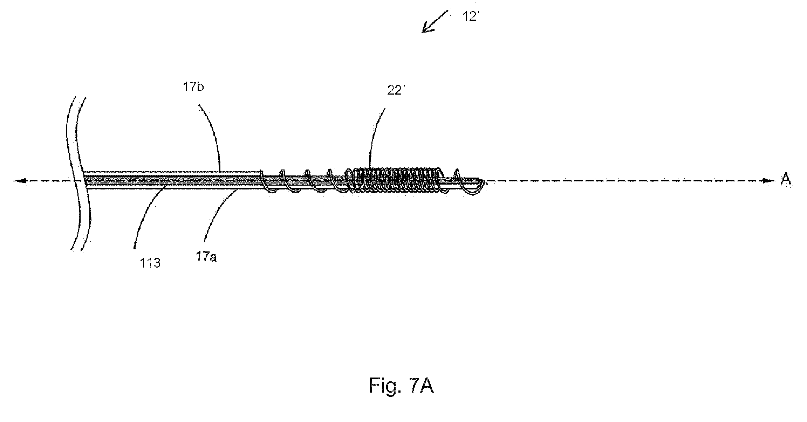

An example of an electrical ablation element including an electrically conductive element with a resistive part 22, suitable for any of the embodiments described herein, is shown in Figure 7. In this embodiment, apparatus includes an elongate insulated mandril 13 to support the electrically conductive element 12 and the resistive part 22 includes a resistive coil wound around a distal tip of the mandril 13. In this embodiment, the coil has a first distal end and a second proximal end with the distal end at the distal end of the mandril 13, although in other embodiments, the ends of the coil do not have to be at proximal and distal longitudinal positions, but can be anywhere along the length of the resistive part 22. Structurally, the coil can be larger than the resistive part and include the resistive part as part of the coil. However, in this embodiment, the resistive part is the whole coil. In this embodiment, the distal tip of the mandril is tapered, although this is not necessary in all embodiments. The taper may provide flexibility to make the distal end, or the whole electrical ablation element, easier to track within a body vessel. Additionally, the mandril 13 may have a curvature at the distal tip to facilitate tracking.

The coil may make good contact with a vessel wall during use.

The conductive element includes a first conductive isolated wire 17a, preferably copper, coupling the first end of the coil to the first power supply coupling zone 14 and a second conductive isolated wire 17b, also preferably copper, coupling the second end of the coil to the second power supply coupling zone 15 to form a closed loop or circuit along the path created by the control unit, the first wire 17a, the coil 22, and the second wire 17b. However, in other embodiments, the first and second conductive isolated wires do not need to be coupled to the ends of the coil, but can be coupled at any points along the coil. Furthermore, in some embodiments the first and second wires do not need to be insulated.

The first and second wires 17a, 17b may have an insulator disposed about their outer surface to isolate or insulate them.

In this embodiment the mandril 13 extends the longitudinal length of an elongate section of the apparatus, the elongate section 21 including the section to be inserted into a patient. The longitudinal length of the elongate section of the apparatus in this embodiment is about 1.5 metres, and the diameter of the apparatus in the elongate section is preferably in the region of 0.5 to 1 mm.

In Figure 7, a battery is shown in place of the control unit 40. However, in preferred embodiments, the control unit 40 as described herein is used.

Figure 7A shows an embodiment similar to that shown in Figure 7 although only the distal end is shown in Figure 7A.

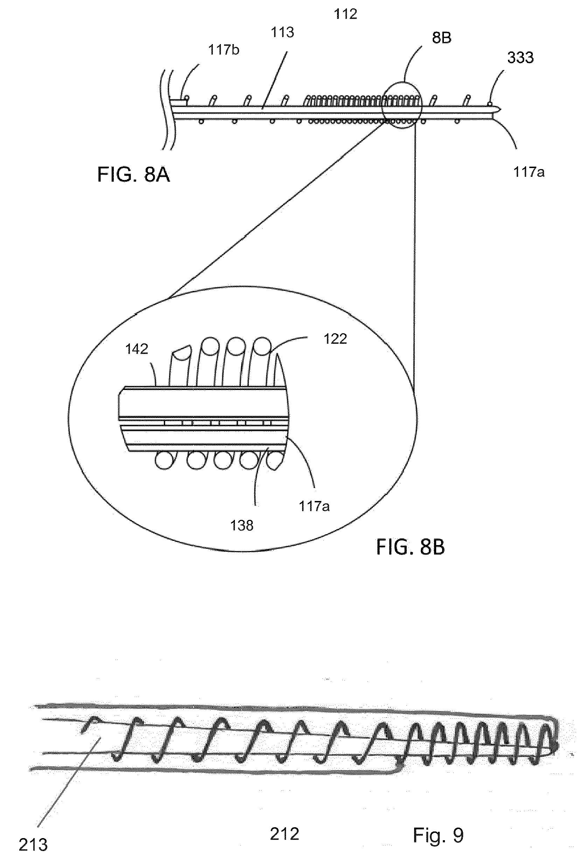

Figs. 8A-B depict details of an electrical ablation element similar to that of Fig. 7 although only the distal end is shown in Figure 8A. The features of Figs. 8A and 8B can equally be used for the element of Fig.7. Fig. 8A shows a cross-sectional view of the ablation element 112. Fig. 8B shows a blown-up view of the element around circle 8B. In Fig. 8A, the mandril 113 has a uniform outer diameter from the proximal end to the distal end. The first and second wires may have an insulator disposed about their outer surface to electrically insulate or isolate them from the rest of the assembly. Insulator 138 is disposed about the first wire 117a in Fig. 8B. The coil 122 may be electrically insulated or isolated from the rest of the assembly, for example by having an insulator, such as a PTFE polymer coating, disposed about its outer surface.

Additionally, the ablation element 112 may have a shrink tubing. For example, the shrink tubing may extend around the mandril 113, the first wire 117a, the second wire 117b, and/or any other portion of the ablation element. As one advantage, the shrink tubing may immobilize or bind the mandril 113, the first wire 117a, and the second wire 117b in place so that they are immobilized relative to each other. The shrink tubing may also immobilize and/or extend over a part of the coil 122 to keep the coil 122 in position as the assembly is in use. Alternatively or additionally in Fig. 8B, the shrink tubing 142 may only be disposed about the mandril 113. In this configuration, the shrink tubing 142 may act to isolate or insulate the mandril 113 from the rest of the assembly.

The first wire 117a may be attached via a first lead and/or conductive wire to the control unit. Additionally, the second wire 117b may be attached to the control unit through a second lead and/or conductive wire. Various conductive wires and connectors may be used to electrically couple parts of the ablation element.

Figure 9 shows a view of a distal end of another ablation element 212 in many ways similar to that of Fig. 7 and Fig. 8A and 8B but in which the density of coil turns increases from the proximal end to the distal end of the mandril 213.

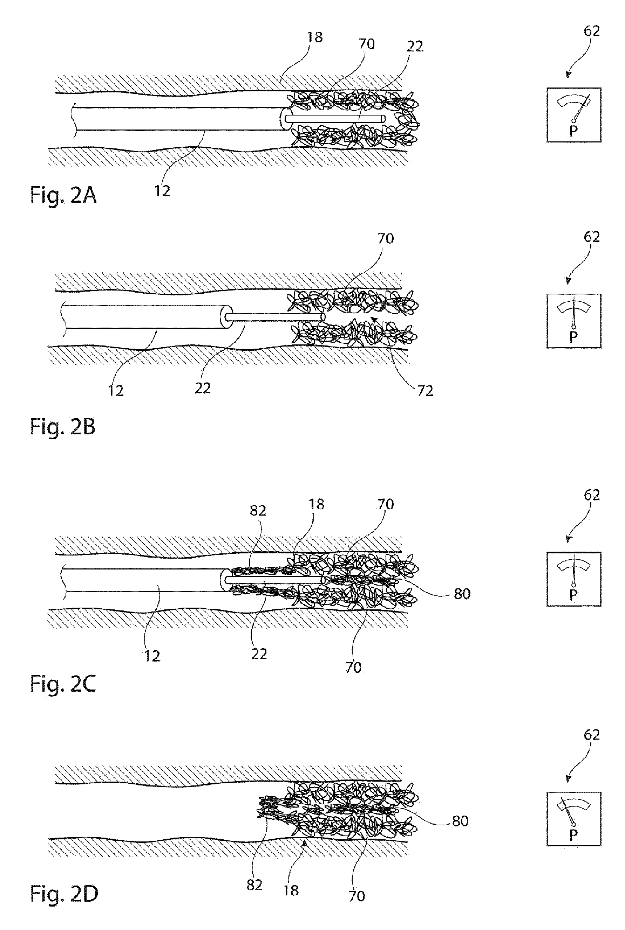

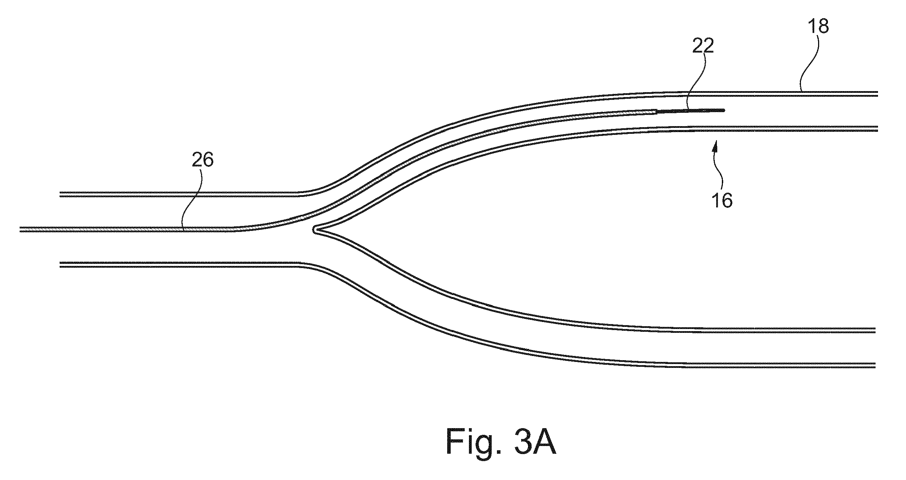

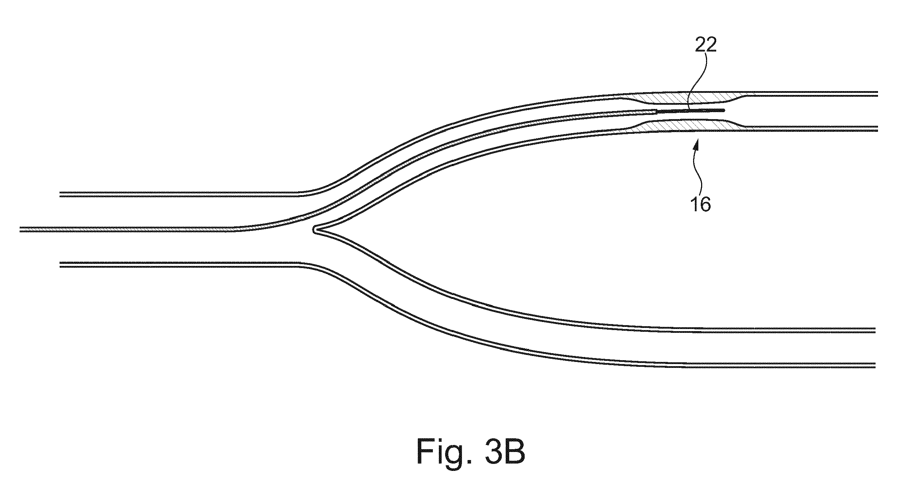

The apparatus 10 of Figure 1A is intended to cause heating of blood within the vessel 18 and the vessel 18 itself so as to occlude the vessel by a combination of heat induced vessel contraction and formation of a blood clot. Figures 2A to 2D and 3A to 3F show in schematic form the preferred mode of operation of the apparatus 10 of Figure 1A and method of ablating a vessel 18, although the vessel contraction is shown more clearly in Figures 3B to 3F than in Figures 2A to 2D.

Referring first to Figure 3A, this shows the resistive part 22 in position at the desired treatment site 16 in a vessel 18.

Referring to Figure 3B, once the resistive part 22 has been positioned at the desired location in the vessel 18, the control unit 40 commands the power unit 44 to supply energy to the resistive part 22, as a result of which current passes from the first power supply coupling zone 14, via the resistive part 22, to the second power supply coupling zone 15, causing the resistive part to heat. The heating of the resistive part 22 causes the vessel wall to contract due to heat damage of the vessel wall.

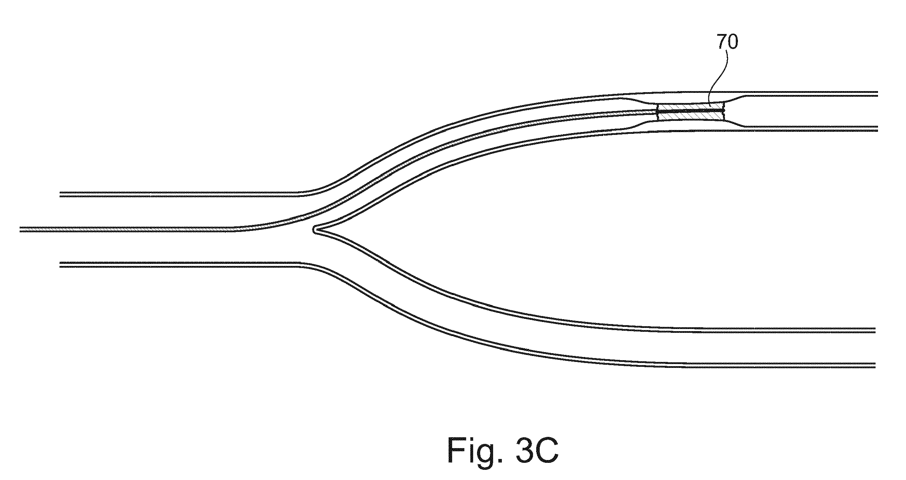

Referring to Figures 2A and 3C, further heating of the resistive part 22 as a result of current passing through the resistive part causes local heating of blood and as a result coagulation of the blood to form a clot 70 around the exposed resistive part 22.

This phase of the operation of the apparatus 10 preferably occurs at a first power level which is relatively high, indicated as such in the depiction of the meter display 62 in Figure 2A.

The progress of ablation in this first phase is preferably controlled by one or more sensors, for instance by means of a temperature probe 24 and temperature sensor unit 46 in the control unit 50. In practice, sensing temperature will aim to detect an increase in temperature indicative of passing a threshold at which a blood flow obstruction is formed, for example at which blood will coagulate to form a clot 70 and/or at which the vessel will contract. It will be noted that although in preferred embodiments the first phase creates a blood flow obstruction including both vessel contraction and blood coagulation, other embodiments can create a blood flow obstruction in the first phase using just vessel contraction or just blood coagulation.

It is not to be excluded that the control unit 50 could be operated without sensors, for example for a predetermined period of time at a predetermined energy which is considered sufficient to create a blood flow obstruction of the required dimensions. This is, though, not preferred as it is preferable to have as precise as possible an indication of the actual state of clotting of blood and/or vessel contraction within the vessel 18 and therefore of the occlusion which is formed.

Referring now to Figure 2B, at the end of the first phase of Figures 3B, 3C and 2A, that is once a blood flow obstruction, in this example including clot 70, is determined to have been formed in the vessel 18, the electrical ablation element 12 including the electrically conductive element, in particular the resistive part 22, is partially retracted, such that the resistive part 22 is at least partially exposed outside the blood flow obstruction or blood clot 70. The withdrawal of the resistive part 22 will generally leave an aperture or lumen 72 within the blood flow obstruction or blood clot 70. In the example shown in Figure 2B, the resistive part 22 is only partially removed from within the volume of the blood flow obstruction or blood clot 70, although in other embodiments the resistive part 22 may be fully retracted from the blood flow obstruction or blood clot 70 so that the distal end of the resistive part 22 lies close to the blood flow obstruction or blood clot 70 but not therewithin. In one example, a resistive part of around 10mm or so may be retracted by 2 to 10 mm at the end of the first phase of the process.

In this embodiment, during retraction the control unit 50 commands the power unit 44 to apply energy at a second power level lower than the first power level applied during the first phase. In some embodiments, during the retraction process, the control unit 50 may command the power unit 44 to apply no power, that is until after the partial retraction of the resistive part 22.

It will be appreciated that the retraction of the resistive part 22, by means of retraction of the electrically conductive element 12, may be effected manually by the medical practioner or automatically by means of a drive unit 50 provided in or coupled to the control unit 40. A suitable drive unit will be apparent to someone of average skill in the art.

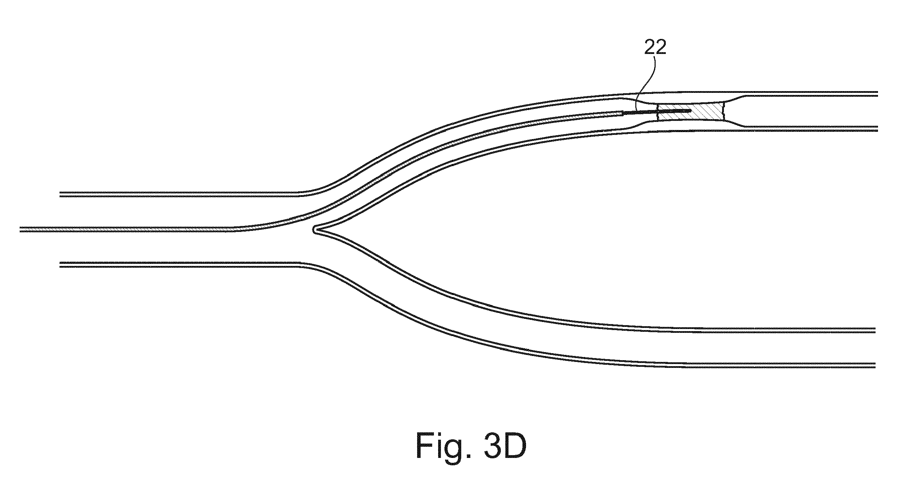

Referring now to Figures 2C and 3D, during a second phase of the ablation process with the resistive part stationary, the control unit 40 commands the power unit 44 to apply energy at the second, lower, power level, which causes ablation or embolization of blood within the vessel 18. In particular, this phase of operation of the apparatus 10 causes blood within the lumen 72 to coagulate and thereby form a blood clot 80 within the lumen 72 so as to close off the lumen 72, as well as further relatively minor blood coagulation around the exposed part of the resistive part 22 to form clotted blood 82 therearound. As the system is operated at a lower power level than the first power level, in this embodiment, the resistive part heats less and the volume of the blood which is clotted is substantially less than the initial blood flow obstruction and preferably such as not to alter notably the length of the blood flow obstruction produced in the first phase of the process shown in Figure 2A. Therefore, the occluding barrier preferably remains substantially the same length even in this second phase of the ablation process. Specifically, the secondary blood clotting 82 is insufficient to fill the width of the vessel 18 and in practice will not obstruct any side vessels. The effective length of the blood flow obstruction will remain the length of the primary blood flow obstruction or blood clot 70.

The degree of clotting during the second phase of the ablation process of Figure 2C and 3D can again be sensed by the temperature probe 24 and temperature sensor unit 46. It will be appreciated that the temperature reached during this second phase may very well differ from those experienced during the first phase of the process, depicted in Figure 2A. The person skilled in the art will be able to determine suitable thresholds for achieving such secondary clotting 80, 82.

The process of partial retraction and ablation or embolization at a lower power level depicted in Figure 2C and 3D can be carried out a plurality of times during the process. In one example, the resistive part 22 may be retracted only a small distance from within the blood flow obstruction or blood clot 70, the second phase carried out, the resistive part 22 retracted a little further, the second ablation or embolization phase operated again and so on, until it has been deemed that a sufficient secondary barrier 80 has been produced within the lumen 72 of the first formed blood flow obstruction. In other examples, the second phase may be carried only once.

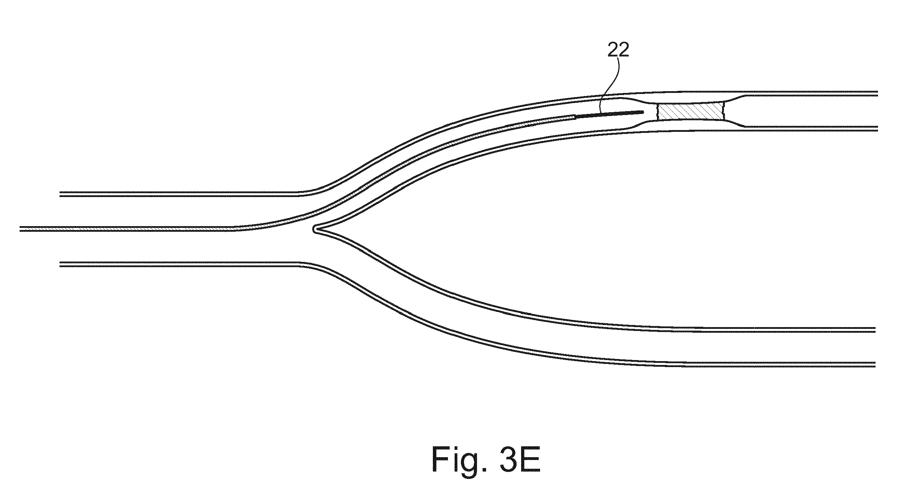

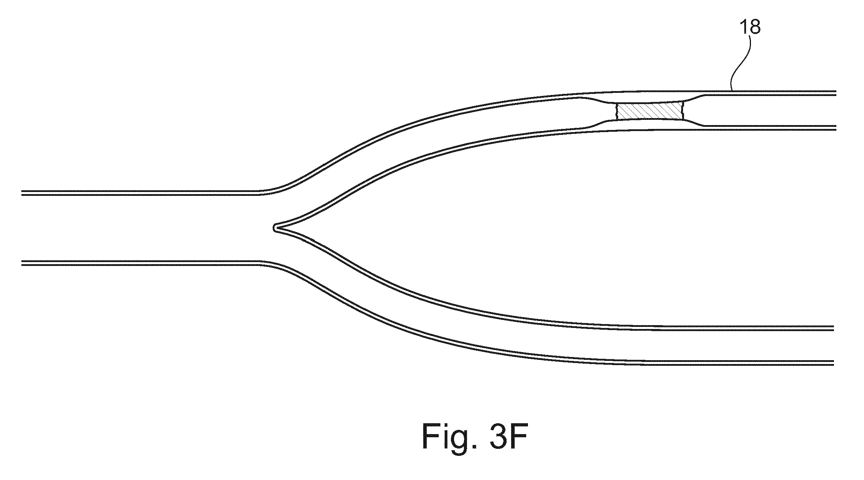

At the end of the second ablation or embolization phase, the electrical ablation element including the electrically conductive element and consequently the resistive part 22 are completely retracted from the treatment site and the vessel 18, as shown in Figure 3E, thereby to leave a blood flow obstruction formed of a vessel contraction, a first blood clot 70 and secondary blood clots 80, 82. At this stage the control unit 50 will have commanded the power unit 44 to cut all power to the resistive part 22. As can be seen in Figure 3F, there is produced a blood flow obstruction, including vessel contraction and a blood clot formation 70-82, which is completely sealed and which, moreover, is not unduly long.

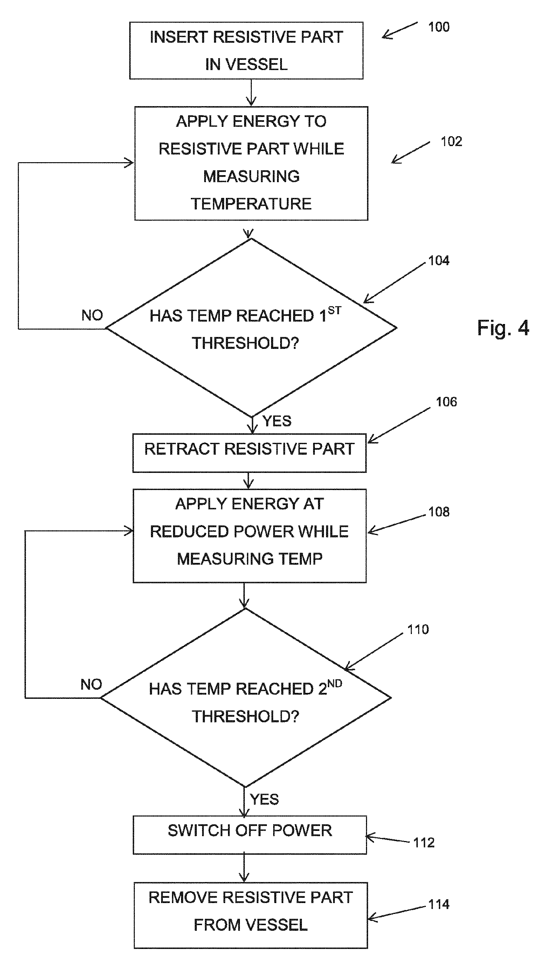

Figures 4 to 6 are flow charts depicting three different modes of operation of the apparatus 10 of Figure 1A, carried out by the control unit 40. The skilled person will appreciate that the apparatus 10 can be operated also in modes other than those described in connection with Figures 4 to 6. The skilled person will also appreciate that the control unit 40 may be set up to operate in a single mode of operation, but that in other embodiments may be programmed to operate in one of a plurality of modes of operation, as desired by the practitioner or required for the medical indication.

Referring first to Figure 4, in this mode of operation, the electrical ablation element including the electrically conductive element 12 is, at step 100, inserted endoluminally into a patient's vessel up to the treatment site. Once positioned as necessary, at step 102, energy is applied to the resistive part 22, preferably at the first power level, while measuring the temperature of the resistive part 22. At step 104, the control unit 40 and in particular the processing unit 42, by continuous monitoring of temperature, determines whether the temperature has reached a first threshold indicative of generation of flow obstruction, which may include a blood clot 70. If the threshold has not been reached, the control unit 40 continues commanding the power unit 44 to apply energy to the resistive part at the first energy level. On the other hand, if at step 104 it is determined that the first threshold has been reached, the resistive part is retracted, either to remain partially within the obstruction or clot formation 70 or to be fully withdrawn therefrom as described above, and the processing unit 42 then commands at step 108 the power unit 44 to apply energy to the resistive part 22 at reduced power, with the resistive part stationary and while continuing to measure temperature. The processing unit 44 continues monitoring the temperature at step 110 to determine therefrom whether this has reached a second threshold level. If the second threshold is deemed not to have been reached at step 110, the system continues operating in accordance with step 108, that is to apply energy at the reduced power level. On the other hand, if it is determined at step 110 that the second threshold has been reached, the processing unit 42 switches off power at step 112, on the basis that the condition at Figure 2C is deemed to have been reached. At step 114, the electrical ablation element including the electrically conductive element 12 and therefore the resistive part, is removed from the vessel.

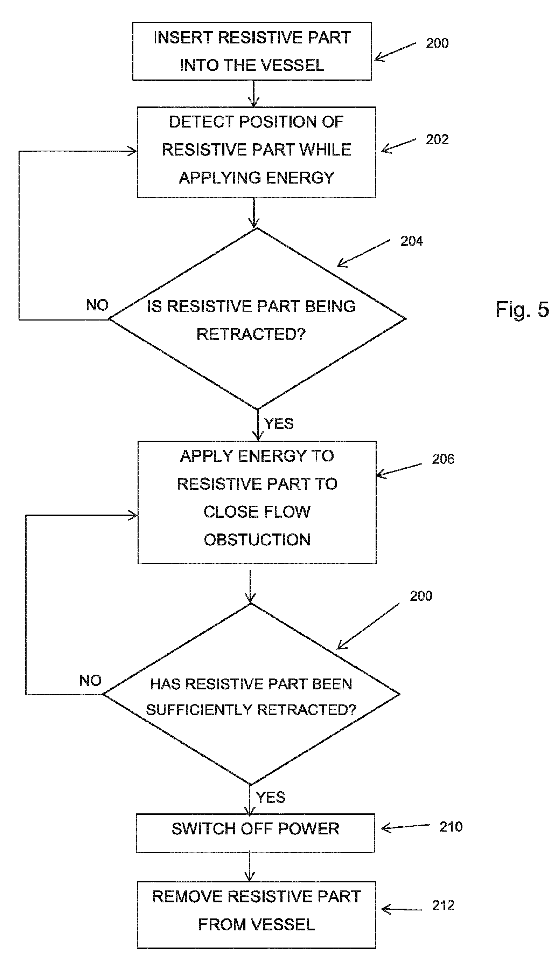

In the embodiment of Figure 5, a different mode of operation is shown. At step 200, the electrical ablation element including the electrically conductive element 12, and therefore resistive part 22, are inserted endoluminally into the vessel, as in step 100 of the embodiment of Figure 4. At step 202 the processing unit 42 detects the position of the resistive part within the vessel while applying energy preferably at the first power level. If at step 204 it is determined that the resistive part is being retracted, operation proceeds to step 206. On the other hand, if it is not detected that the resistive part is being retracted, the process returns to step 202 to continue applying energy while continuing to monitor the position of the resistive part. When it is determined that the resistive part is being retracted, the process proceeds to step 206 at which energy continues to be applied to close the blood flow obstruction or thrombus formation. This energy is in the preferred embodiment applied at the lower energy level in order to create secondary thrombus formations as shown in Figure 2C. In other embodiments energy can be applied at the first power level or a power level sufficient to cause greater, that is larger, thrombus formation and/or vessel contraction and further occlusion of the vessel.

The processing unit 42 continues to monitor the position of the resistive part 22 and when it is determined that it has been retracted by a predetermined distance deemed sufficient to close the blood flow obstruction or thrombus formation 70, the processing unit 42 moves to step 210, at which it commands the power unit 44 to switch off power to the resistive part 22. Then, at step 212, the electrical ablation element including the electrically conductive element 12 and therefore the resistive part 22 are removed from the vessel.

In its simplest form, the embodiment of Figure 5 does not measure changes in temperature during the ablation process and the system continues applying energy where it is determined that the resistive part is being retracted. This embodiment, therefore, in its simplest form avoids leaving an open lumen 72 within the formed blood flow obstruction or blood clot 70, which could as a result lead to incomplete occlusion of the vessel 18 and risk of recanalization. It is to be understood that the embodiment of Figure 5 could also include steps to measure temperature and to determine whether these have reached the first and/or the second thresholds disclosed above in connection with the embodiment of Figure 4.

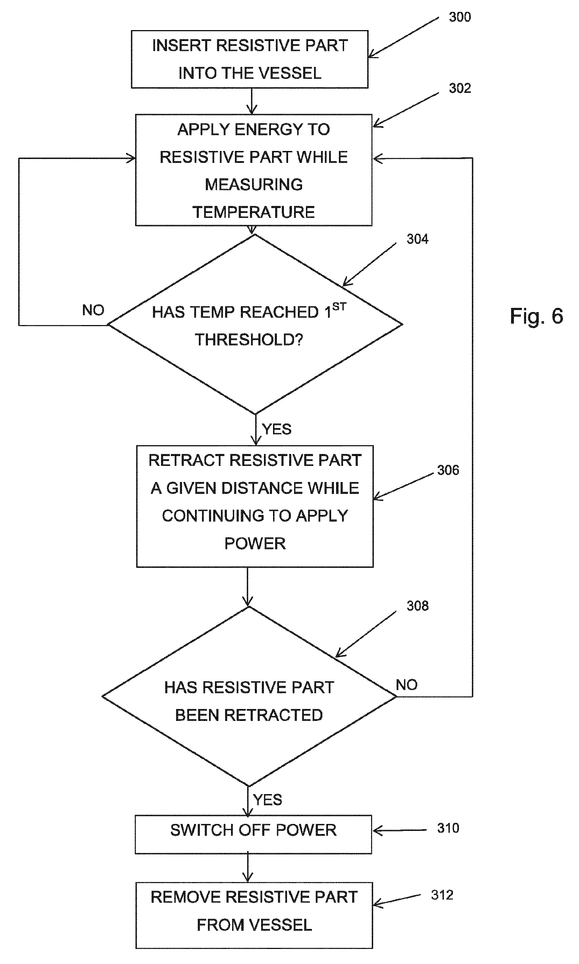

Another embodiment of mode of operation of the apparatus 10 of Figure 1 A is shown in Figure 6. In this embodiment, as with the previous embodiments, at step 300 the electrical ablation element including the electrically conductive element 12 and therefore resistive part 22 are introduced into the vessel to the location at which treatment is to be carried out.

At step 302 the control unit 42 commands the power unit 44 to apply energy to the resistive part 22, preferably at the first power level, whilst at the same time measuring temperature at the resistive part 22. At step 304, the processing unit 42 determines whether the temperature has reached the first threshold and if it has not, step 302 continues to operate. On the other hand, when it is determined at step 304 that the first threshold has been reached, operation passes to step 306, at which the control unit commands retraction of the resistive part 22 by a given distance. This may be by operating the drive unit 50 shown in Figure 1A or by displaying on a user interface a command instructing the user to effect the necessary withdrawal (the user can equally be warned by an acoustic and/or vibratory command). The control unit 42 may operate in a continuous manner, such that during the retraction by the given distance the control unit applies power as shown in Figure 6. This power can be at the first power level, or at the lower second power level. In other embodiments, the control unit 42 may operate in a discontinuous manner and apply no power during the retraction by the given distance. At step 308 the processing unit 42 determines whether the resistive part 22 has been retracted by a desired distance. This distance is deemed to be the desired total length of the occluding barrier formed by the blood clot 70. If it is deemed that the resistive part has not been retracted by the desired distance, the process returns to step 302 to continue applying energy, preferably at the first power level and with the resistive part stationary. It is to be understood that in returning to step 302 vessel contraction and/or a blood clot of the type shown in Figure 2A will be generated, but more proximally, in effect to extend the length of the blood flow obstruction, which may include extending the length of blood clot 70, to create a longer occluding barrier extending radially from the resistive part 22 to the vessel walls. Once, at step 308, it has been determined that the resistive part has been retracted by the desired distance, typically equivalent to the desired length of the blood flow obstruction or blood clot 70, the process passes to step 310, at which power is switched off and then, at step 312, the electrical ablation element including the electrically conductive element 12 and resistive part 22 are removed from the patient.

It is to be understood that between steps 308 to 310, the control unit 40 and in particular the processing unit 42 may command a process equivalent to steps 108 and 110 in the embodiment of Figure 4 in order to create a secondary occlusive barrier 80, 82 in order to close off any lumen in the formed blood flow obstruction or blood clot 70. It is also to be understood that a phase of reduced power may be effected between steps 306 and 308 of the embodiment of Figure 6, that is after retraction by the given distance with the resistive part preferably stationary, to create a progressive barrier 80 within the lumen 72 of the formed blood flow obstruction or blood clot 70. In such an event, the embodiment of Figure 6 will then apply power at a higher power level over a longer period of time in order, in effect, to grow the blood clot 82 at the exposed end of the resistive part 22 and/or contract the vessel until the blood flow obstruction is deemed to have filled the volume of the vessel 18, that is extending from the resistive part to the vessel walls.

Thus, the embodiment of Figure 6 can be used to create an occlusion of varying lengths as well as ensuring closure of the lumen left by the retracting resistive part 22.

Although the methods of operation are described predominantly in connection with the embodiment shown in Fig. 1A, they can be used for any of the described devices.

Furthermore, although temperature thresholds are used in above described methods as prompting a subsequent step of the method, the detection of other operational changes can be used instead or in addition. For example, the electrical impedance, for example relative to a patient, the inductance, capacitance, and/or resistivity of the resistive part can be measured by an appropriate sensor provided for example in the control unit. Embodiments can include a pressure sensor at the resistive part coupled to a pressure sensing unit in the control unit to measure the pressure at the resistive part of the ablation element. In addition, in embodiments that include a drive unit for retracting the ablation element, the resistance to retraction can be measured as an indication of the adhesion of the resistive part, for example to the target site, to elements of or in the vessel such as the blood flow obstruction. A change in any of these measured parameters for example to above or below a predetermined threshold, can be determined to be indicative of an operational change suitable for prompting the subsequent step of the method. In addition, the rate of change of any of the measured parameters can be used to determine such an operational change, for example if the rate of change is above or below a predetermined threshold. Appropriate thresholds can be determined by the skilled person.

All optional and preferred features and modifications of the described embodiments and dependent claims are usable in all aspects of the invention taught herein. Furthermore, the individual features of the dependent claims, as well as all optional and preferred features and modifications of the described embodiments are combinable and interchangeable with one another.

The disclosures in UK patent application 1517822.1, from which priority is claimed, and in the abstract accompanying this application are incorporated herein by reference.

## Claims

1. Apparatus for closing a blood vessel including:
an electrically conductive element for being passed endoluminally to a treatment site, the electrically conductive element having a proximal section and a distal section, having first and second power supply coupling zones at the proximal section, and having a resistive part positionable at the distal section, wherein the electrically conductive element electrically couples the first and second power supply coupling zones via the resistive part;
a power supply for coupling to the first and second power supply coupling zones for supplying energy to the resistive part;
a detection unit for detecting at least one operational change at the resistive part;
a control unit coupled to the power supply and to the detection unit; wherein the control unit is operable to control the power supply to supply energy to the resistive part at a first power level, to detect at least one operational change at the resistive part and to control the power supply to supply energy to the resistive part at a second power level lower than the first power level once the operational change has been detected.

2. Apparatus according to claim 1, including a user notification unit coupled to the control unit, the control unit being operable to generate a notification on detection of the operational change in the resistive part.

3. Apparatus according to claim 1 or 2, wherein the control unit is operable to command a partial retraction of the resistive part of the electrically conductive element in a proximal direction on detection of the operational change.

4. Apparatus according to claim 3, including a positioning unit coupled to the control unit, the positioning unit being operable to effect said partial retraction of the resistive part in a proximal direction on detection of said operational change.

5. Apparatus according to claim 3, wherein the control unit is operable to generate a notification to effect said partial retraction of the resistive part.

6. Apparatus according to any preceding claim, including a resistive part position sensor coupled to the control unit, wherein the control unit is operable to control the power supply to supply energy to the resistive part at the or a second power level lower than the first power level when partial retraction of the resistive part has been detected.

7. Apparatus according to claim 6, wherein the control unit is operable to command the power supply to supply power to the resistive part until the resistive part has been retracted by a predetermined distance; wherein the predetermined distance is preferably equivalent to a desired length of closure of the vessel.

8. Apparatus according to any preceding claim, wherein the apparatus is configured to cause a blood coagulation in a vessel when operated at the second power level.

9. Apparatus for closing a blood vessel including:
an electrically conductive element for being passed endoluminally to a treatment site, the electrically conductive element having a proximal section and a distal section, having first and second power supply coupling zones at the proximal section, and having a resistive part positionable at the distal section, wherein the electrically conductive element electrically couples the first and second power supply coupling zones via the resistive part;
a power supply for coupling to the first and second power supply coupling zones for supplying energy to the resistive part;
a position sensor arranged to detect the position of the resistive part in a patient;
a control unit coupled to the power supply and to the position sensor;
wherein the control unit is operable to control the power supply to supply energy to the resistive part at a first power level, to determine movement of the resistive part by a first predetermined distance and to apply power to the resistive part when the resistive part has been deemed to have been moved by the first predetermined distance.

10. Apparatus according to claim 9, wherein the control unit is operable to apply power to the resistive part continuously to cause a blood flow obstruction, provided at least in part by a blood coagulation in a vessel, while the resistive part is moved by the predetermined distance.

11. Apparatus according to any one of claims 9 to 10, wherein the control unit is operable to apply power to the resistive part up to a second distance greater than the first predetermined distance; wherein the second distance is preferably equivalent to a desired length of closure of the vessel.

12. Apparatus according to any one of claims 9 to 11, including a detection unit for detecting at least one operational change at the resistive part, the detection unit being coupled to the control unit, wherein the control unit is operable to control the power supply to supply energy to the resistive part at a second power level lower than the first power level once the operational change has been detected.

13. Apparatus according to any one of claims 9 to 12, wherein the resistive part has a longitudinal length in a longitudinal direction of the apparatus and the first predetermined distance is less than said longitudinal length.

14. Apparatus according to any one of claims 9 to 13, including a positioning unit coupled to the control unit, the positioning unit being operable to move the resistive part in at least a proximal direction.
